# EUROPEAN PATENT APPLICATION

(11) **EP 3 124 578 A1**
(43) Date of publication of application: **01.02.2017**
(21) Application number: 15768501.7
(22) Date of filing: 30.03.2015
(51) Int. Cl.: C10L 1/00

(54) **PROCESS AND DEVICE FOR PRODUCING FUEL HYDROCARBON OIL**

(30) Priority: 28.03.2014 JP 2014069467
(71) Applicant: Royal Corporation Co., Ltd., Tokyo 105-0014 (JP); Saegusa, Eiji, Yokohama-shi, Kanagawa 234-0055 (JP)
(72) Inventor: KATO, Atsushi, Okazaki-shi, Aichi 444-3446 (JP)
(74) Representative: Denison, Christopher Marcus
(86) International application number: PCT/JP2015/059966
(87) International publication number: WO 2015/147322

(57) **Abstract**

Provided are a method and a device with which carbon dioxide is used as a starting material to yield methanol, without requiring high-temperature highpressure conditions or the addition of hydrogen, and fuel hydrocarbons such as gas oil or heavy oil are produced in a satisfactory yield. The method is a method for increasing the amount of a hydrocarbon oil, characterized by: mixing methanol with water into which air has been bubbled in the presence of a catalyst; mixing the resultant liquid mixture with the feed hydrocarbon oil to produce an emulsion; and contacting this emulsion with a gas or aqueous solution which contains carbon dioxide.

## Description

### Technical Field

The present invention relates to a production method and a production apparatus for producing a hydrocarbon oil.

### Background of the Invention

Recently, the problem of global warming due to the carbon dioxide emission has become serious. In the industrial world, efforts for low emission and the development of alternative energy have been made and certain effects have been achieved. Meanwhile, the nuclear power generation affected by the Great East Japan Earthquake is practically ceased at present, accordingly fossil fuel has to be used more than ever, and thus the emission of carbon dioxide is accelerated.

Today, meanwhile, there is a problem of environmental destruction caused by energy. It is known that fossil fuel contains a sulfur component, a nitrogen component and a phenol component which generate hazardous gas leading to environmental destruction. It is also an urgent task to take a measure against global warming caused by the generation of CO₂. In order to prevent or suppress the environmental destruction, it is effective to reduce the generation of hazardous gas which is generated by use of fossil fuel and to improve the fuel efficiency.

As an invention for improving the fuel efficiency of fuel hydrocarbon from such a viewpoint, there is an invention described in Patent Literature 1 performed by the present inventor. The invention disclosed in Patent Literature 1 is an invention in which an active water prepared by mixing a natural plant composite enzyme in water is allowed to react with an oil, and the reacted water also functions as a fuel due to the hydrolysis reaction of the material oil by an enzyme; thus the invention can improve the fuel efficiency, easily suppresses the generation of hazardous substances, provides a production method and a production apparatus for producing a stable fuel oil, and can contribute to the prevention of the environmental destruction.

### Prior Art Document

### Patent Document

Patent Document 1: Japanese Patent Laid-Open No. 2012-72199

### Summary of the Invention

### Problems to be solved by the Invention

However, recently, there have been demanded techniques capable of drastically solving the problem of global warming due to the carbon dioxide emission and the problem of alternative energy due to the depletion of fossil fuel. It has already been clarified that fuel hydrocarbons such as gasoline fraction can be produced from the reaction between hydrogen and carbon dioxide; however, reactions under conditions of high temperature and high pressure are required. For a method for synthesizing a hydrocarbon by using carbon dioxide as a material, without using hydrogen as a material, various attempts as implementation of artificial photosynthesis have been performed. However, fuel hydrocarbons have not yet been efficiently produced.

The present invention, made in light of the above-described problems, has an object of providing a method and an apparatus in which methanol is produced by using a carbon dioxide-containing gas or liquid as a material, without needing the conditions of high temperature and high pressure and without addition of hydrogen, and further fuel hydrocarbons such as light oil and heavy oil are produced in good yields.

### Means for solving the problems

The present inventor made a diligent study in order to solve the above-described problem, and consequently succeeded in the production of a hydrocarbon oil increased in the amount than the hydrocarbon oil used as a material by mixing a methanol-containing water, a hydrocarbon oil (fuel hydrocarbon), and a carbon dioxide-containing gas or aqueous solution, at normal temperature without being pressurized.

Specifically, the present invention is as follows.

The method for increasing the amount of a hydrocarbon oil according to the present invention is a method in which water bubbled with air in the presence of a catalyst and methanol are mixed, the obtained liquid mixture and a material hydrocarbon oil are mixed to prepare an emulsion, and the emulsion and a carbon dioxide-containing gas or aqueous solution are brought into contact with each other.

The production method for producing a hydrocarbon oil according to the present invention is a production method in which water bubbled with air in the presence of a catalyst and methanol are mixed, the obtained liquid mixture and a material hydrocarbon oil are mixed to prepare an emulsion, the emulsion and a carbon dioxide-containing gas or aqueous solution are subjected to a contact treatment, and a hydrocarbon oil is collected from the resulting treated product.

In the above-described method for increasing the amount of a hydrocarbon oil or the above-described production method for producing a hydrocarbon oil, the hydrocarbon oil is produced on the basis of the following formulas (1) and (2) :

CO₂ + H₂O + CₙH₂ₙ₊₂ = Cₙ₊₁H₂ₙ₊₄ + 3/2O₂ (1)

CH₃OH + CₙH₂ₙ₊₂ = Cₙ₊₁H₂ₙ₊₄ + H₂O (2)

Here, in the above-described method for increasing the amount of a hydrocarbon oil and the above-described production method for producing a hydrocarbon oil, the catalyst is preferably an enzyme and/or a zeolite or a zeolite-like substance (the catalyst may be a substance supported on a zeolite or a zeolite-like substance), water preferably contains an active species, and the hydrocarbon oil is preferably any one of light oil, heavy oil, heating oil, kerosene, and gasoline.

Moreover, the production apparatus for producing a hydrocarbon oil according to the present invention is an apparatus including: a mixing tank for mixing water bubbled with air in the presence of a catalyst and methanol; an emulsion preparation tank for preparing an emulsion by mixing the liquid mixture contained in the mixing tank and a hydrocarbon oil; and a contact tank for bringing the prepared emulsion and carbon dioxide into contact with each other.

Here, the contact tank is preferably a tank in which the emulsion is exposed to carbon dioxide in the atmosphere, or carbon dioxide recovered from any one of the tanks or a carbon dioxide recovery apparatus or the like, or a carbon dioxide-containing gas or aqueous solution is introduced into the emulsion preparation tank.

### Effects of the Invention

According to the present invention, the amount of a fuel hydrocarbon can be increased by at least 10% by mixing methanol-containing water, the fuel hydrocarbon, and carbon dioxide. Accordingly, the present invention can produce a fuel hydrocarbon to be an energy source by using carbon dioxide regarded as a cause for global warming as a material, thus can achieve a similar effect as the use of the carbon in the atmosphere as energy by plants through fixing the carbon in the atmosphere by photosynthesis, and can solve both of the energy problem and the environmental problem regarded as the problems of the whole of the earth by the mankind.

### Brief Description of the Drawings

[Figure 1] Figure 1 is a structural view of an active water production apparatus for producing active water.
[Figure 2] Figure 2 is a structural view of a uniformly mixing apparatus.
[Figure 3] Figure 3 is a structural view showing a structure of a stirring tank.
[Figure 4] Figure 4 illustrates a structure of a stirrer.
[Figure 5] Figure 5 is a vertical cross-sectional view showing the inside of the stirrer.
[Figure 6] Figure 6 illustrates a structure of a pulse filter and a structure of a precision filter.
[Figure 7] Figure 7 is a vertical cross-sectional view of a Newton's separation tank.
[Figure 8] Figure 8 is a vertical cross-sectional view showing a stirrer in another example.

### Detailed Description of the Invention

The present invention is directed to the increase of a fuel hydrocarbon as follows: methanol-containing water and a fuel hydrocarbon are mixed into an emulsified state, the methanol/water/fuel hydrocarbon mixed solution in an emulsified state and a carbon dioxide-containing gas or aqueous solution are brought into contact with each other, and thus the amount of the fuel hydrocarbon is increased. The fuel hydrocarbon obtained by the method of the present invention is increased in the amount thereof, as compared with the material fuel hydrocarbon, at least by 10%, preferably by 20%, more preferably by 30%, and furthermore preferably by as large as 40%. For example, the proportion of the increased amount of the produced fuel hydrocarbon is 10% to 100%, preferably 10% to 50% and more preferably 30% to 50% of the amount of the material fuel hydrocarbon.

Accordingly, the present invention allows a fuel hydrocarbon to be produced extremely efficiently.

The present invention includes the following steps (i) to (iii).
(i) A water treatment step of activating water by bubbling water with air in the presence of a catalyst.
(ii) A step of preparing an emulsion by mixing the activated water, a hydrocarbon oil, and methanol.
(iii) A step of bringing the emulsion and carbon dioxide into contact with each other.

Here, instead of performing the mixing step after the water treatment step, and further subsequently performing the reaction step, the water treatment step and the mixing step can also be performed simultaneously, the mixing step and the reaction step can be performed simultaneously, and moreover, the water treatment step, the mixing step, and the reaction step can all also be performed simultaneously.

In the present invention, the reactions in the above-described steps (i) to (iii) are represented by the following formulas (1) and (2).

CO₂ + H₂O + CₙH₂ₙ₊₂ = Cₙ₊₁H₂ₙ₊₄ + 3/2O₂ (1)

CH₃OH + CₙH₂ₙ₊₂ = Cₙ₊₁H₂ₙ₊₄ + H₂O (2)

The above-described formula (1) involving carbon dioxide can be represented by the following chain reactions.

CO₂ + 2H₂O → CH₃OH + 3/2O₂ (3)

CH₃OH + CₙH₂ₙ₊₂ → Cₙ₊₁H₂ₙ₊₄ + H₂O (4)

The water used is preferably allowed to be in a state of including active species (such as active oxygen and a hydroxyl radical). In order to allow water to include active species, hydroxyl radical is generated in water, or alternatively in the water treatment step, an enzyme such as lipase is mixed in water, or a zeolite or a zeolite-like substance is included in the reaction system. In addition, water is preferably bubbled with air for a predetermined time of period. For example, in order to activate water so as for the reactions represented by the above-described formulas (1) and (2) to be performed at normal temperature, the bubbling is preferably performed at normal temperature for 72 hours; however, depending on the state of the water as a material, the bubbling time may be appropriately altered. The mixing step is preferably performed so as for the reaction mixture to be uniform.

In the present invention, a hydrocarbon oil means a substance mainly composed of a hydrocarbon and exhibiting a liquid state under at normal temperature and normal pressure (for example, a temperature of 15°C and a pressure of 1 atm), and represented by CₙH₂ₙ₊₂ or Cₙ₊₁H₂ₙ₊₄ (chain saturated hydrocarbon). Here, n is, for example, 1 to 40, and preferably 1 to 20. Examples of such a hydrocarbon oil include, without being limited to: heavy oil, light oil (for example, n = 10 to 20), gasoline (for example, n = 4 to 10), naphtha, kerosene (for example, n = 10 to 15), heating oil (for example, n = 9 to 15), and the like.

In the present description, a hydrocarbon oil is also referred to as a "fuel hydrocarbon" or a "fuel oil." Similarly, the water bubbled with air in the presence of a catalyst may be also referred to as an "enzyme water" or "water."

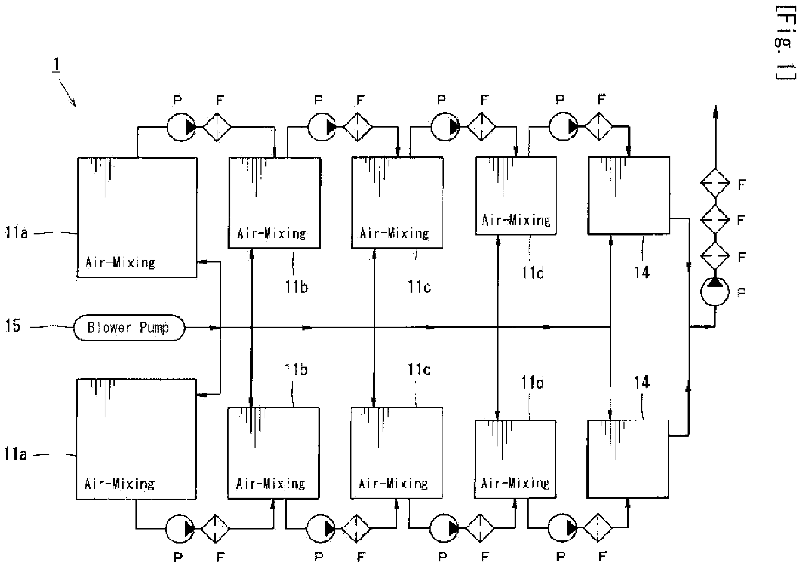

The production method for producing a fuel hydrocarbon according to the present invention can be implemented by utilizing, as the production apparatus thereof, the fuel production apparatus disclosed in Japanese Patent Laid-Open No. 2012-72199. In other words, Figure 1 is a structural view of a water treatment apparatus 1 for activating water. The water treatment apparatus 1 includes a plurality of mixing tanks 11 (11a to 11d), stabilizing tanks 14, a blower pump 15 for sending air to each of the tanks, pumps P for transferring a liquid between the tanks, and filters F for removing impurities during the transfer. The mixing tanks 11a to 11d are provided in two systems as shown in top and bottom parts of the figure, and the mixing tanks 11a, 11b, 11c, 11d in each of the systems are connected in series by the pumps P and the filters F.

To a mixing tank 11a, for example, soft water and a zeolite or a zeolite-like substance containing an enzyme powder (for example, EP-10) are fed in a proportion of 1000 liters and 500 g; the soft water, the enzyme powder and zeolite are stirred and mixed for, for example, 72 hours by the air supplied from a blower pump 15. The ratio of the water and the zeolite or zeolite-like substance containing an enzyme powder is preferably, for example, approximately 0.05% (weight ratio) of an enzyme powder with respect to 99.95% (weight ratio) of the soft water. The origin of the enzyme constituting the enzyme powder is not particularly limited; the enzyme may be derived from animal, plant, or microorganism. Preferably, the enzyme contains lipase as a main component, and more preferably is formed of lipase and cellulase. It is more preferable that the enzyme contains 98% (weight ratio) of lipase and 2% (weight ratio) of cellulase. Such an enzyme can be extracted from fruits such as mango, avocado, pineapple, noni, sea buckthorn, and the like. Commercially available enzymes are also be utilized. Preferably, the enzyme powder is obtained by thermally drying such an enzyme and used with improved preservability.

The resultant water mixture is transferred to the next mixing tank 11b by the pump P after a certain time period. During the transfer, impurities are removed by the filter F. In the mixing tank 11b, the water mixture are again stirred and mixed by air supplied from the blower pump 15. This cycle is repeated until being finished in the mixing tank 11d, the water mixture is transferred from the mixing tank 11d to the stabilizing tank 14, and then alcohol is added to the water mixture in the stabilizing tank 14. This alcohol may be, for example, methanol or ethanol, and methanol is preferable. A preferable ratio of the alcohol is, for example, about 5% to 20% (weight ratio) of methanol with respect to the water mixture.

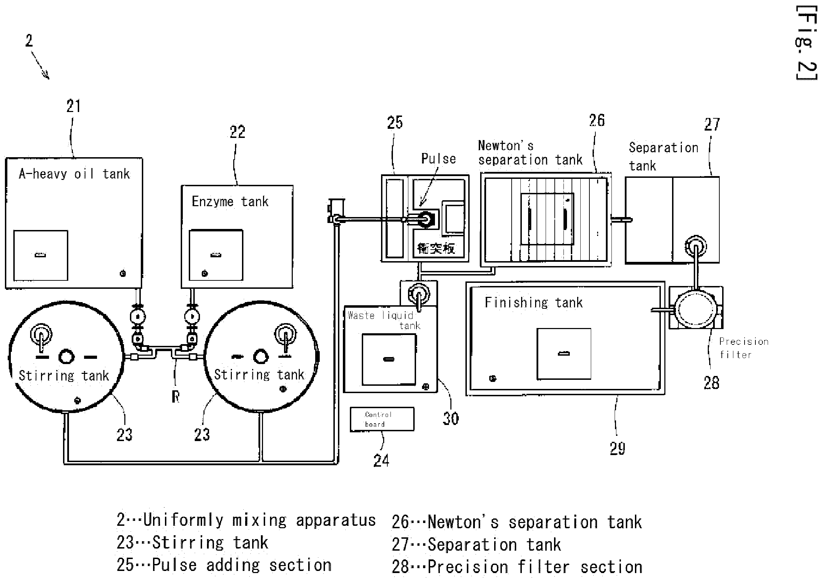

The active water refined with alcohol is taken out from the stabilizing tank 14 by the pump P. During this process, impurities are removed by three filters F. The refined active water which is taken out is transferred to a different appropriate container or stored in an active water tank 22 of a uniformly mixing apparatus 2 shown in FIG. 2.

In the present embodiment, the water activated by the water treatment apparatus 1 is activated so as to perform the reactions of the reaction formulas (1) and (2) even at normal temperature when a material oil is added in the reaction step.

FIG. 2 is a structural view of the uniformly mixing apparatus 2. The uniformly mixing apparatus 2 includes a material oil tank 21 as an oil storage section for storing oil, an active water tank 22 as an active water storage section for storing active water, two stirring tanks 23, a control board 24, a pulse adding section 25, a Newton's separation tank 26, a separation tank 27, a precision filter section 28, a finishing tank 29, and a waste liquid tank 30.

The material oil tank 21 is a tank for storing oil used as a material, and feeds the material oil stored therein by a unit of necessary amount into the stirring tanks 23 through a pipe R. The material oil may be, for example, A-heavy oil, B-heavy oil, C-heavy oil, light oil, kerosene or the like. In the present embodiment, A-heavy oil is used.

The active water tank 22 is a tank for storing active water refined by the water treatment apparatus 1, and feeds the active water stored therein by a unit of necessary amount into the stirring tanks 23 through the pipe R.

The stirring tanks 23 are each a tank for producing fuel oil by stirring and thus reacting the fed material oil (for example, A-heavy oil) with the active water. The reaction here is a hydrolysis reaction of the material oil by the enzyme. The ratio of the material oil and the active water to be fed into the stirring tanks 23 may be appropriately adjusted in accordance with the type of the material oil. For example, a preferable ratio is 60% of A-heavy oil and 40% of the active water, 70% of light oil and 30% of the active water, or 70% of kerosene and 30% of the active water.

The control board 24 is a control section for controlling the elements of the apparatus, and executes various types of controls, for example, turns power supply on or off. The pulse adding section 25 vibrates the fuel oil produced in the stirring tanks 23 so that residue is easily removed. The residue is, for example, water which was left unreacted, impurities in the heavy oil, or the like.

The Newton's separation tank 26 stores the fuel oil and drops the residue downward by the force of gravity, and thus extracts the fuel oil remaining in an upper part thereof.

The separation tank 27 further separates the residue from the fuel oil. The precision filter 28 removes the residue from the fuel oil by a filter. The finishing tank 29 stores the produced refined fuel oil. The waste liquid tank 30 stores a waste liquid generated in the pulse adding section 25 and the Newton's separation tank 26.

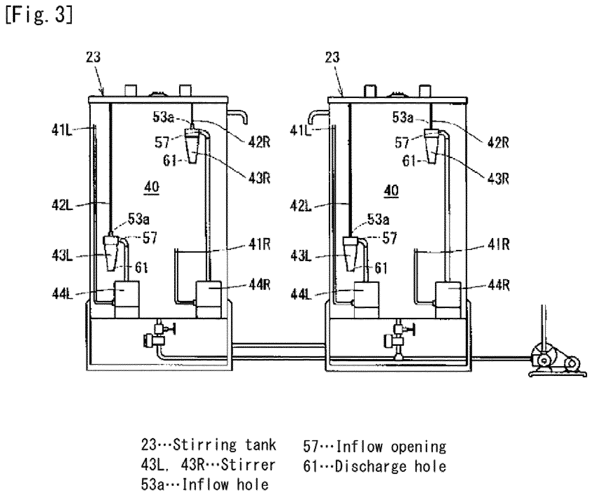

FIG. 3 is a structural view showing a structure of the stirring tanks 23. The stirring tanks 23 each include a generally cylindrical stirring space 40. In the stirring space 40, stirrers 43 (43L, 43R) and pumps 44 (44L, 44R) are provided. Among the stirrers 43, the stirrer 43L shown left in the figure is provided in a lower part of the stirring space 40, and the stirrer 43R shown right in the figure is provided in an upper part of the stirring space 40. Thus, the stirrers 43 are located discretely up and down and also discretely left and right. The stirrers 43 are respectively connected to the pumps 44 (44L, 44R). From the pumps 44, fuel oil, active water, or an active water-oil mixture is supplied.

The pump 44L is connected to a pipe having a suction opening 41L in an upper part thereof. The pump 44L feeds the fuel oil, active water, or active water-oil mixture to the stirrer 43L, and thus generally uniformly circulates the fuel oil, active water, or active water-oil mixture in the stirring space 40.

The pump 44R is connected to a pipe having a suction opening 41R in a lower part thereof. The pump 44R feeds the fuel oil, active water, or active water-oil mixture to the stirrer 43R, and thus generally uniformly circulates the fuel oil, active water, or active water-oil mixture in the stirring space 40. As each of the pumps 44L and 44R, it is preferable to use a 30 to 40 atmospheric pressure pump.

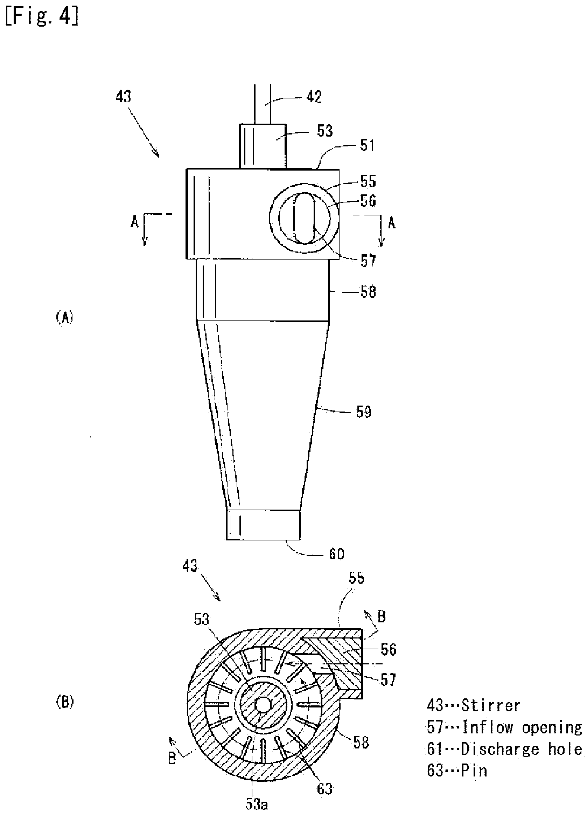

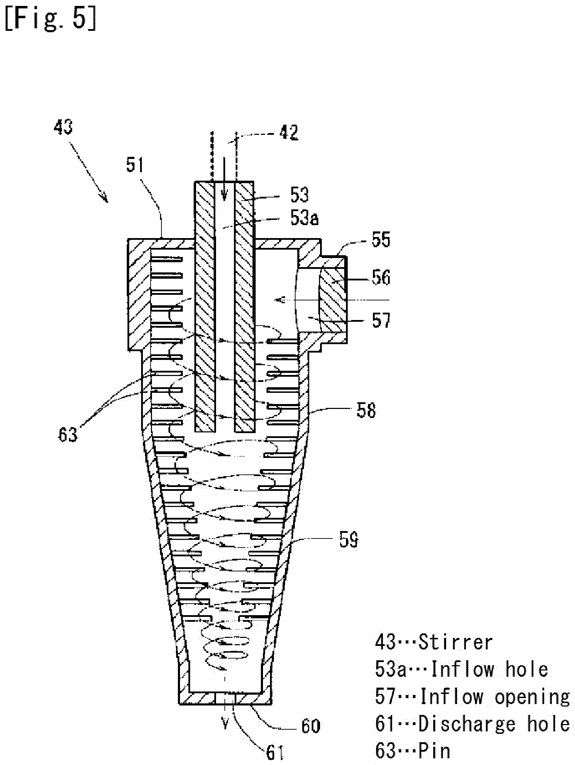

FIG. 4 is an explanatory drawing of a structure of the stirrer 43. The stirrer 43 is a hollow metal element, and mainly includes a generally cylindrical head 51, an inverse conical body 59 which are continuous from the head 51, and a trailing end section 60 below the body 59. On a central part of an upper surface of the head 51, a cylindrical central shaft 53 is provided. The central shaft 53 has an inflow hole 53a (see FIG. 5) running throughout the central shaft 53 in an up/down direction. The fuel oil, active water, or active water-oil mixture flows to the inside through the inflow hole 53a.

The head 51 has an inflow opening 57 in a part of a side surface thereof, through which the fuel oil, active water, or active water-oil mixture flows to the inside. The inflow opening 57 is a hole running from the outside to the inside of the head 51, and is surrounded by a cylindrical connection cover 55. The connection cover 55 has a thread groove 56 in an inner surface thereof, such that the pipe connected to the pump 44 is attachable thereto.

As shown in FIG. 4(B), which is a cross-sectional view of FIG. 4 (A) along line A-A, the position of the inflow opening 57 and the orientation of the connection cover 55 are such that the fuel oil, active water, or active water-oil mixture flows toward the inner surface in an eccentric manner with respect to the center of the stirrer 43. Owing to this, the fuel oil or the like flowing to the inside through the inflow opening 57 efficiently rotates around the cylindrical central shaft 53 as the rotation axis.

As shown in FIG. 5, which is a cross-sectional view of FIG. 4(B) taken along line B-B, a plurality of pins 63 stand on an inner surface of the stirrer 43. The plurality of pins 63 are located with an interval so as not to cross each other. For example, 55 to 80 pins having a diameter of 0.03 mm may be provided at an interval of about 10 mm.

In the trailing end section 60 of the stirrer 43, a discharge hole 61 is provided. The stirrer 43 thus structured can stir oil and active water efficiently to cause a decomposing reaction. In more detail, the fuel oil, active water, or active water-oil mixture flowing to the inside through the inflow opening 57 rotates around the central shaft 53 while moving toward the discharge hole 61 with the rotation radius thereof being gradually decreased in a whirlwind manner. During this process, the fuel oil, active water, or active water-oil mixture is stirred by the plurality of pins 63 provided in the stirrer 43. By the rotation in a whirlwind manner, a negative pressure is generated in a lower part of the central axis 53, which allows the fuel oil, active water, or active water-oil mixture to flow to the inside through the inflow opening 53a. Namely, the stirrer 43L shown in FIG. 3 mainly takes in the oil, suctioned from the suction opening 41L, by the pump 44L through the inflow opening 57, and mainly takes in the active water through the inflow opening 53a, and stirs the oil and the active water. By contrast, the stirrer 43R mainly takes in the active water, suctioned from the suction opening 41R, by the pump 44R through the inflow opening 57, and mainly takes in the oil through the inflow opening 53a, and stirs the oil and the active water. Owing to the stirrer 43, the active water and the oil can collide against each other to be stirred in the water having a high pressure, and thereby the reaction of the above reaction formula (1) can be promoted.

While being stirred in the stirring tank 23 having the stirrer 43 for a predetermined time period (for example, about 15 to 20 minutes), the oil and the active water stirred while moving in a whirlwind manner in the stirrer 43 contact each other 300 to 500 times. Thus, the hydrolysis reaction is promoted, and the molecules become smaller and the specific gravity is decreased.

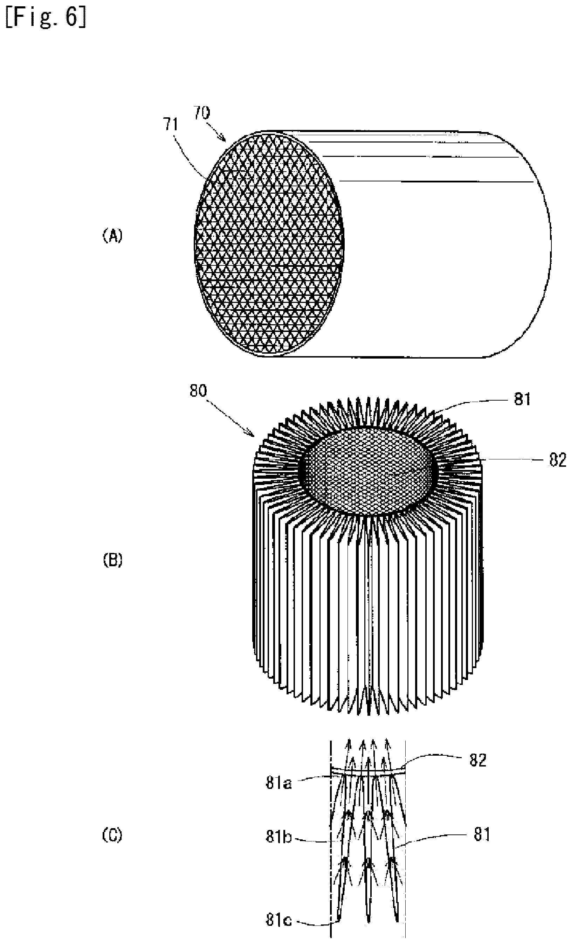

FIG. 6 (A) is a perspective view of a pulse filter 70 provided in the pulse adding section 25. The pulse filter 70 is provided between two line mixers, and allows the fuel oil to pass through holes formed in lattice partitions 71. The pulse adding section 25 (especially, the partitions 71) is formed of a sintered ceramic material.

The partitions 71 are slowly twisted inside like a screw, and vibrates the fuel oil flowing to the inside to promote the reaction. Owing to this, the fuel oil is put into a state where impurities are easily removed.

FIG. 6(B) is a perspective view of a precision filter 80 provided in the precision filter section 28. The precision filter 80 includes a cylinder-like element 82 formed of a mesh material and a filter 81 provided around the cylinder-like element 82. The filter 81 radially expands from the center thereof. The fuel oil is allowed to pass through the filter 81 from outside toward the cylinder-like element 82, and thus impurities can be removed.

The filter 81 is provided radially. As shown in FIG. 6(C), which is a partial enlarged view thereof, the fuel oil can pass the entirety of a plate-like face 81b of the filter 81 from a base-side end 81a to a tip-side end 81c. Therefore, even when impurities are accumulated at the base-side end 81a and it becomes difficult for the fuel oil to pass through the filter 81, the plate-like face 81b allows the fuel oil to pass with no problem and to remove the impurities.

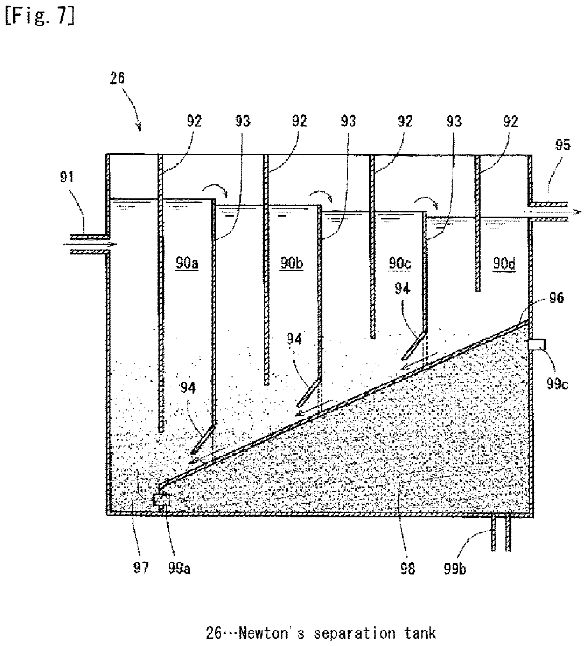

FIG. 7 is a vertical cross-sectional view of the Newton's separation tank 26. The Newton's separation tank 26 mainly includes an inclining plate 96 provided in the vicinity of a bottom section, and a plurality of higher-level plates 92 and a plurality of lower-level plates 93 which are alternately provided above the inclining plate 96. The Newton's separation tank 26 has a liquid inflow opening 91 for the connection with an element on the previous stage and a liquid discharge opening 95 for the connection with an element on the subsequent stage. A space is provided between lower ends of the higher-level plates 92 and the inclining plate 96, so that the fuel oil can move forward and rearward. Upper ends of the lower-level plate 93 are at a lower position than those of the higher-level plates 92, so that the stored fuel oil overflows and moves into the next storage section. The lower-level plates 93 each have a movable plate 94 at a lower end thereof, and a lower end of the movable plate 94 contacts the inclining plate 96. The higher-level plates 92 and the lower-level plates 93 are arranged alternately in this order, and are provided such that lower ends thereof are gradually at a higher position along the inclination of the inclining plate 96.

Owing to this structure, the fuel oil flows to the inside through the liquid inflow opening 91 into a first storage section 90a. Impurities thereof are accumulated in a lower part thereof, and the refined fuel oil is stored in an upper part thereof and overflows into a second storage section 90b next to the first storage section 90a. This process is repeated from the first storage section 90a to a fourth storage section 90d, and the resultant clean fuel oil is discharged from the liquid discharge opening 95.

The impurities precipitated in the storage sections 90a to 90d move downward along the inclining plate 96. For this process, the movable plates 94 are opened to allow the impurities to move downward. The movable plates 94 are not opened in the opposite direction and so the impurities do not flow in the opposite direction.

The impurities moved downward along the inclining plate 96 are transferred from a recovery opening 97 to a recovery section 98 via a valve 99a and are recovered in the recovery section 98. The valve 99a is opened and closed intermittently. When the residue is stored in a certain amount, the valve 99a is opened to allow the residue to be recovered in the recovery section 98 and then is closed. Then, air is discharged via an air discharge valve 99c provided in an upper part of the recovery section 98. The impurities recovered in the recovery section 98 may be taken out via a recovery valve 99b and discarded.

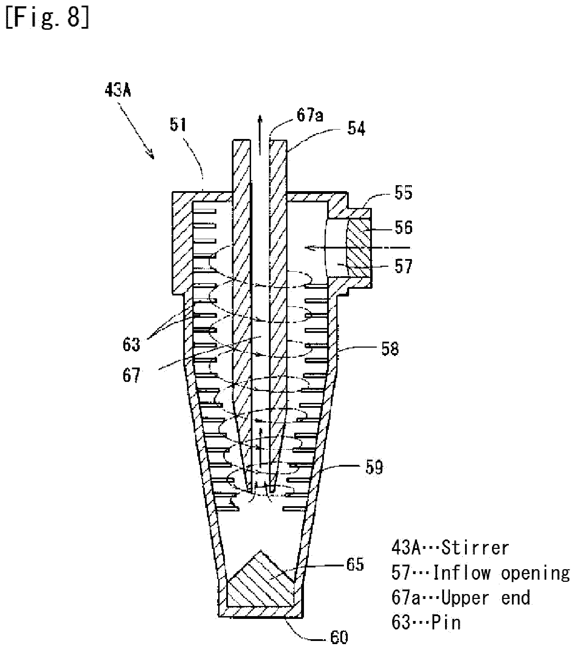

As the stirrer 43, a different type of stirrer 43A as shown in Figure 8 may also be used. The stirrer 43A does not have a discharge hole in the trailing end section 60, and includes a central pipe 54 instead of the central shaft 53 provided in the embodiment described above. The central pipe 54 has a cylindrical shape having a hollow section 67 inside, and an upper end 67a of the hollow section acts as a discharge opening for the fuel oil. In the stirrer 43A having such a structure, active water and oil flowing to the inside through the inflow opening 57 rotate while moving downward with the rotation radius thereof being gradually decreased in a whirlwind manner, and then move from a lower end to the upper end of the central pipe 54 and are discharged from the upper end. The stirrer 43A also provides the same function and effect as those of the stirrer 43 described in the above-described embodiment. A fuel oil can be produced by performing the reactions represented by the reaction formulas (1) and (2), by using the above-described water treatment apparatus 1, uniformly mixing apparatus 2 and Newton's separation tank 26. Hereinafter, the present invention is described more specifically by way of Examples. However, the present invention is not limited to these Examples.

### Example 1

### Production Test of Fuel Oil

The present inventor performed a production test of a fuel hydrocarbon by using the above-described apparatuses. The test used the apparatuses described in the above-described embodiment for carrying out the invention, and adopted the methods described in the same embodiment.

### <Test Results>

In the present test, light oil was used as a material oil, and as a new oil, a fuel prepared by mixing the material oil and methanol/active water (mixing volume ratio was 370:150) was able to be obtained.

In the present test, the fed substances are as follows.

| | Specific gravity | Volume (liters) | Mass (kg) |
|---|---|---|---|
| Light oil | 0.845 | 3364.30 | 2842.83 |
| Enzyme water | 1 | 7057.10 | 7057.10 |
| Methanol | 0.79 | 412.60 | 325.95 |
| Total | | 10834.00 | 10225.89 |

Compared with this, the total of the overflowed new fuel and the residual amounts in the respective tanks is as follows.

| | Specific gravity | Volume (liters) | Mass (kg) |
|---|---|---|---|
| Light oil | 0.845 | 4923.92 | 4160.71 |
| Methanol enzyme water | 0.975 | 5587.96 | 5448.26 |
| Total | | 10511.88 | 9608.97 |

Consequently, the balance of the light oil was +1317.88 kg, the methanol enzyme water balance was -1934.80 kg, the total balance was -616.92 kg, and the increment rate of light oil reached as large as 46.36%.

### Example 2

### Additional Test

A similar additional test performed after Example 1 also yielded the following data.

### [Product due to Additional Test 1]

Total substance balance -16.0 kg
Component substance balances
   Light oil 126.5 kg
   Water -117.1 kg
   Methanol -25.3 kg
Increment rate of light oil 40.2%

### [Product due to Additional Test 2]

A continuous 9-batch (370 liters of light oil in a batch) production test was performed.
Fed light oil: 2813.85 kg
Produced light oil: 3826.88 kg
Increment rate of light oil: 36%

### [Product due to Additional Test 3]

### Fed amount (total: 9768.1 kg)

Preparation of Newton's tank
Methanol enzyme water (load cell measured value: 7036.0 kg, methanol concentration: 0.786%)
(Items: enzyme water (6980.7 kg), methanol (55.3 kg), light oil (none))
Total amount fed in 1 course (6 batches)
Methanol enzyme water: 868.6 kg ((i) 145.3, (ii) 144.0, (iii) 145.6, (iv) 143.6, (v) 145.2, (vi) 144.9) kg
Light oil: 1863.5 kg (2218.45 l, specific gravity: 0.840) ((i) 310.3, (ii) 310.3, (iii) 311.1, (iv) 310.3, (v) 311.3, (vi) 310.2) kg
(Fundamental preparation: enzyme water 130 kg/130 l, methanol 25.7 kg/32.5 l, light oil 319.2 kg/380 1)
Recovered amount (total: 9608.6 kg)
After completion of the total course of 1 course (6 batches), the masses were measured by manual operations.

### Methanol enzyme water: 7092.7 kg

Light oil: 2515.9 kg (2995.12 liters,
specific gravity: 0.84)
Mass balance (-159.5 kg)
Fed amount - recovered amount = 9768.1 kg - 9608.6 kg = -159.5 kg

According to the measurement value of the load cell, the total weight was found to be -140 kg, and hence the substantial mass increase or decrease was approximately 19.5 kg.

Increase or decrease of methanol enzyme water: -811.9 kg (fed amount 7904.6 kg - recovered amount 7092.7 kg)

Increase or decrease of light oil: +652.4 kg (fed amount 1863.5 kg - recovered amount 2515.9 kg, 776.6 liters}
Light oil increment rate: 35.0%

### Example 3

### Verification of Test Results

On the basis of the substance balance data of the above-described test results, the carbon source of the light oil increased in amount was verified.

First, by verifying the carbon source of the amount increase of the light oil based on the substance balance data, the following points were clarified from the substance balance data obtained by the production of the new fuel.
1) The decreased amount of methanol alone was not able to elucidate the increased amount of light oil, and thus it was clarified that another carbon source was involved.
2) The decrease of the amount of water was clearly observed, and hence a carbon source other than methanol is required; accordingly, it is necessary to consider that a chemical reaction formula (1) (the involvement of carbon dioxide in the reaction consumes water) holds; thus, the carbon dioxide in the air may be safely considered to be a carbon source for the amount increase of light oil.

From the foregoing discussion, a carbon source other than methanol is required, and the involvement of carbon dioxide in the reaction consumes water, and hence it has been shown that the carbon dioxide in the air can be a carbon source for the amount increase of light oil. The result that the production rates of light oil in Examples 1 and 2 are different from each other may be ascribable to the conditions that as can be seen from the formulas (1) to (4), water is a material for the amount increase of light oil, and the production rate is varied, for example, depending on the nature (pH, hardness, turbidity and others) of the water; however, foregoing Examples have verified that approximately 30% or more of the amount increase is possible.

### Example 4

### Verification of Carbon Source

In order to investigate whether or not carbon dioxide was a carbon source for the amount increase of light oil, a verification of the introduction of a stable carbon isotope 13C into the hydrocarbon in the light oil was performed according to the following procedures.

### <Experimental Procedures>

1) A PET bottle (vessel 1) having an internal volume of 500 ml was filled with CO₂ labeled with 13C and sealed tightly.
2) In the vessel 1, 100 g of a liquid mixture (liquid temperature: 30.5°C) flowing out from a pulse tank was placed and the vessel was sealed tightly.
3) The vessel was shaken for approximately 5 seconds and then allowed to stand still. During the shaking, it was verified that the vessel 1 was crushed.
4) After 6 days from the completion of the above-described operations, the stable carbon isotope ratio δ13C was measured by using the DELTA V ADVANTAGE of Thermo Fischer Scientific K.K.

This measurement was performed at Isotope Research Institute, Inc. (in Yokohama City Industry-Academia Colloborative Research Center, 1-1-40 Suehiro-cho, Tsurumi-ku, Yokomama, CEO: Akira Hanawa).

According to the results of two runs of analysis (analysis 1 and analysis 2), the stable carbon isotope ratio (δ13C vs PDB) was -26.4(%) in the analysis 1 and -27.8(%) in the analysis 2.

In other words, the result shows that the δ13C of the light oil brought into contact with 13C was higher by 1.4% than the δ13C of the original light oil. This shows that the content of 13C was higher as compared with the original light oil. Consequently, carbon dioxide was demonstrated to be a carbon source for the amount increase of light oil.

As described above, the substance balance in the production of the new fuel and the reaction test using the stable carbon isotope 13C demonstrated that as the carbon source for the amount increase of light oil, not only methanol but also carbon dioxide is involved.

In other words, when the carbon of the carbon dioxide in the atmosphere was used as the carbon source, the carbon was able to be artificially fixed, and it was secondarily shown that a fuel (light oil in the above-described examples) is synthesized (increased in amount) by using such carbon. According to the chemical reaction formulas implemented by the present invention, carbon dioxide was taken and oxygen was released, and thus carbon is converted into an energy source in a form of a fuel; accordingly, it is obvious that there occurs a reaction capable of being safely referred to as an artificial photosynthesis. Surprisingly, the reactions based on the above-described reaction formulas (1) and (2) were implemented, in Examples of the present invention, at normal temperature, without applying a high temperature and a high pressure. Of course, the present invention is not limited only to the case where the reactions based on the reaction formulas (1) and (2) are all performed at normal temperature. For example, by performing some steps under warming or under pressure, in a further shorter time, a high performance methanol or a fuel hydrocarbon can also be produced.

In above-described Examples, carbon dioxide was allowed to react with a fuel (light oil); however, the carbon taken in according to the reaction formula (1) can be utilized as methanol, accordingly, the methanol can be processed into materials of various chemical products or into a fuel itself, and thus the application range of such carbon is wide.

Moreover, recently, a large number of apparatuses for recovering carbon dioxide from burnt gas have been disclosed. At present, the carbon dioxide recovered from such an apparatus is discarded in the earth crust in the deep underground; however, the recovered carbon dioxide can be incorporated into the reaction system of the present invention, thus the effect of carbon circulation is created, combustion is made possible without increasing the amount of carbon dioxide in the atmosphere; in this case, high-concentration carbon dioxide can be allowed to react, and more efficient production of methanol or a fuel can be performed.

From the above-described description, the present invention can be said to be an epochmaking technique also from the viewpoint of the global environment.

Herein, for the stable carbon isotope ratio (δ13C: delta 13C), some additional remarks are presented. In other words, a direct measurement of the absolute concentration an element or an isotope is extremely difficult, and accordingly, a method for measuring the relative abundance ratios of isotopes is generally adopted. The stable isotope ratio δ is represented in terms of permillage (1/1000: permil) by how the stable isotope abundance ratio of a standard substance and the stable isotope abundance ratio of an analysis sample are separated (deviated) from each other. The standard substance of carbon (C) is the belemnite (PDB: Pee Dee Belemnite, the calcium carbonate of the belemnite (a fossil of a squid) produced in the Pee Dee stratum in the North Carolina State, United States of America), the content of 12C being 98.894% and the content of 13C being 1.106%.

### Example 5

### Verification of Example 4

In the description until Example 4, it has been theoretically verified that carbon dioxide is the carbon source for the amount increase of the hydrocarbon oil; however, herein, a test was actually performed on the amount increase due to the presence of carbon dioxide. Because of the structure of the apparatus, the structure cannot perfectly shield the carbon dioxide (for example, in the atmosphere), and hence a test was performed on the behavior in the case where carbon dioxide was compulsorily exposed.

### <Test Procedure 1>

The liquid mixture flowing out from the pulse tank was sampled in an amount of 50 g, and placed in a 500-ml sealed vessel; and the degree of the amount increase was measured under the following conditions. The measurement was performed after 1 hour from the above-described sampling.
(i) The case where the space in the vessel was filled with air (in the usual case, the concentration of carbon dioxide was approximately 300 ppm)
   Amount increase: Approximately 15%
(ii) The case where the space in the vessel was completely filled with carbon dioxide
   Amount increase: Approximately 26%

The amount increase found even in (i) is ascribable to the open structure of the interior of the stirring tank/the pulse tank and the reaction mixture is exposed to the atmosphere, and the reaction of the fed methanol. The difference between (i) and (ii) is the concentration of the exposed carbon dioxide; the large amount increase found in (ii) can be ascribable to the high concentration of the carbon dioxide exposed to the liquid mixture and the thereby accelerated reaction.

From the above-described test, after 1 hour, the difference between the amount increases was able to be verified so as to suggest the shortening of the reaction time by the presence of carbon dioxide, and accordingly, a test was performed on the reaction time difference due to the difference in the concentration of carbon dioxide.

### <Test Procedure 2>

The liquid mixture flowing out from the pulse tank was sampled in an amount of 50 g, and placed in a 500-ml sealed transparent vessel; and the time course of the amount increase was measured under the following conditions.
- The case where the vessel was not sealed, and opened to the atmosphere (in the usual case, the concentration of carbon dioxide is approximately 300 ppm)
   The time for approximately 30% of the amount increase: approximately 22 hours
- The case where the space in the vessel was completely filled with carbon dioxide, and the vessel was sealed
   The time for approximately 30% of the amount increase: approximately 90 minutes

From the above-described test results, the possibility of the shortening of the reaction time by positively exposing carbon dioxide was able to be verified.

### Example 6

By using an oxygen detecting tube, it was qualitatively verified whether or not oxygen was actually generated during the reaction as in the chemical reaction formula (1) CO₂ +H₂O + CₙH₂ₙ₊₂ = Cₙ₊₁H₂ₙ₊₄ + 3/2O₂ showing the generation of oxygen.

### <Test Procedure>

During the operation of the stirring tank, the gas in the upper portion of the tank was sampled, and the oxygen concentration was measured with an oxygen detecting tube. The same measurement was also performed for the atmosphere (background) in the vicinity of the tank.
- The atmosphere (background) in the vicinity of the tank
   Oxygen concentration: Approximately 21%
- The gas in the stirring tank
   Oxygen concentration: Approximately 22.5% to 23%

From the results of the test, the generation of oxygen in the above-described chemical reaction formula (1) was able to be verified.

### Industrial Applicability

The present invention can be utilized for increasing the amounts of various oils such as A-heavy oil, B-heavy oil, C-heavy oil, light oil, kerosene and the like..

### Reference Signs List

1...water treatment apparatus; 2...uniformly mixing apparatus; 23...stirring tank; 25...pulse adding section; 26...Newton's separation tank (contact tank); 27...separation tank; 43L, 43R, 43A stirrer; 63...pin

## Claims

1. A method for increasing the amount of a hydrocarbon oil, wherein water bubbled with air in the presence of a catalyst and methanol are mixed; the obtained liquid mixture and a material hydrocarbon oil are mixed to prepare an emulsion; and the emulsion and a carbon dioxide-containing gas or aqueous solution are brought into contact with each other.

2. A production method for producing a hydrocarbon oil, wherein water bubbled with air in the presence of a catalyst and methanol are mixed; the obtained liquid mixture and a material hydrocarbon oil are mixed to prepare an emulsion; the emulsion and a carbon dioxide-containing gas or aqueous solution are subjected to a contact treatment; and a hydrocarbon oil is collected from the resulting treated product.

3. The method according to claim 1 or 2, wherein the hydrocarbon oil is produced on the basis of the reactions represented by the following formulas (1) and (2):
CO₂ + H₂O + CₙH₂ₙ₊₂ = Cₙ₊₁H₂ₙ₊₄ + 3/2O₂ (1)
CH₃OH + CₙH₂ₙ₊₂ = Cₙ₊₁H₂ₙ₊₄ + H₂O (2)

4. The method according to claim 1 or 2, wherein the catalyst is an enzyme and/or a zeolite or a zeolite-like substance.

5. The method according to claim 1 or 2, wherein water comprises an active species.

6. The method according to claim 1 or 2, wherein the hydrocarbon oil is any of light oil, heavy oil, heating oil, kerosene and gasoline.

7. A production apparatus for producing a hydrocarbon oil comprising: a mixing tank for mixing water bubbled with air in the presence of a catalyst and methanol; an emulsion preparation tank for preparing an emulsion by mixing the liquid mixture contained in the mixing tank and a hydrocarbon oil; and a contact tank for bringing the prepared emulsion and carbon dioxide into contact with each other.

8. The apparatus according to claim 7, wherein the contact tank exposes the emulsion to the carbon dioxide in the atmosphere or the carbon dioxide recovered from any one of the tanks, or introduces a carbon dioxide-containing gas or aqueous solution into the emulsion preparation tank.

9. The apparatus according to claim 7, wherein the contact tank exposes the emulsion to the carbon dioxide recovered by a carbon dioxide recovery apparatus or the like.
